# EUROPEAN PATENT SPECIFICATION

(11) **EP 1 250 358 B1**
(45) Date of publication and mention of the grant of the patent: **16.03.2005**
(21) Application number: 01946869.3
(22) Date of filing: 25.01.2001
(51) Int. Cl.: C07K 14/715, C07K 14/56, C07K 7/06

(54) **THERAPEUTIC PEPTIDES**
THERAPEUTISCHE PEPTIDE
PEPTIDES THERAPEUTIQUES

(30) Priority: 25.01.2000 GB 0001712
(43) Date of publication of application: 23.10.2002
(73) Proprietor: Medarex Inc., Princeton, NJ 08540 (US)
(72) Inventor: TOVEY, Michael, Gerard, F-75005 Paris (FR); EID, Pierre, F-75013 Paris (FR)
(74) Representative: Woods, Geoffrey Corlett
(86) International application number: PCT/GB2001/000287
(87) International publication number: WO 2001/055215

(56) References cited:
- WO-A-93/20187
- US-A- 5 861 258

## Description

The present invention provides therapeutic peptides for use as Type 1-interferon (Type 1-IFN) antagonists, in particular such peptides derived from an extracellular portion of the human Type 1-IFN receptor (IFN-R).

### Background of the invention

The type 1 interferons constitute a family of multifunctional cytokines which mediate communication between cells in higher organisms. They include IFN-β, IFN-ω, IFN-τ and various sub-types of IFN-α. Interferons constitute the body's first line of defence against virus infections and the development of cancer.

However, abnormal production of IFN-α has been reported to be associated with a number of autoimmune diseases including systemic lupus erythematosus (Shiozawa et al., 1992, Arthr. & Rheum., 35, 417), rheumatoid arthritis (Hopkins & Meager, 1988, Clin. Exp. Immunol., 73, 88), type 1 diabetes (Stewart et al., 1993, Science 260, 1942; Huang et al., 1994, Cell 1, 469), psoriasis (Shmid et al.,1994, J. Interferon Res., 14, 229) and multiple sclerosis (Degré et al., 1976, Acta Neurol. Scand., 53, 152). A number of clinical reports have also described the development of the symptoms of autoimmune disease, or the exacerbation of underlying autoimmune disease, in patients treated with recombinant IFN-α2 (see, for example, Wada et al., 1995, Am. J. Gastroenterol., 90, 1366 and Perez et al., 1995, Am. J. Hematol., 49, 365). Furthermore, in AIDS patients a direct correlation has been reported between the level of circulating IFN-α and disease progression (Mildvan et al., 1992, The Lancet, 339, 353). The results of other studies suggest that IFN-α also plays an important role in allograft rejection (Afifi et al., 1985, J. Immunol., 134, 3739) and in graft-versus-host-disease (GVHD) (Cleveland et al., 1987, Cell Immunol., 110, 120).

It has been shown, for example, that treatment of cynomologous monkeys with an anti-IFN-R monoclonal antibody which competively binds with Type 1-IFN to the IFN-R results in a marked increase in skin allograft survival. It has also been shown that treatment of animals with the same antibody together with a subeffective dose of cyclosporin A results in prolonged allograft survival in major histocompatibility class I and II antigen divergent animals. Treatment of cynomologous monkeys with an antibody which competively inhibits IFN binding to the IFN-R, together with a sub-effective dose of cyclosporin A, was additionally found to result in marked inhibition of graft-versus-host disease in animals grafted with allogenic bone marrow from major histocompatibilty class I and class II antigen divergent animals (Tovey et al., 1996, J. Leuk. Biol., 59, 512-517; Benizri et al., 1998, J. IFN & Cytokine Res., 18, 273-284).

Macaques infected with simian immunodeficiency virus (SIV) are considered to be a useful model for the study of host factors which play a role in the development of AIDS. In this animal model, production of IFN-α is observed during primary infection but is insufficient to prevent the establishment of a chronic infection and the development of immunodeficiency. A second phase of IFN-α production in SIV-infected macaques is observed after several months. There is a close correlation between the presence of interferon in this second phase and the loss of CD4+ cells, which accompanies the development of clinical signs of disease. In SIV-infected macaques with high levels of circulating IFN-α, administration of an anti-IFN-R antibody which competively binds to the IFN-R with Type 1-IFN was found to result in a pronounced and prolonged increase in the level of circulating CD4+ cells (Khatissian et al., AIDS & Human Retroviruses, 12, 1273-1278). Hence, Type 1-IFN antagonists are of interest for treatment or prophylaxis of HIV infection as well as a number of other diseases where Type 1-IFN has been indicated to have a role in disease development.

The human IFN-R is a heterodimer composed of two polypeptide chains, IFNAR1 and IFNAR2. The presence of the two chains is required for high affinity binding of Type 1-IFN. The human genes for both IFNAR1 and IFNAR2 have been cloned (Uzé et al., 1990, Cell, 60, 224-234; Novick et al., 1994, Cell, 77, 391-400). Expression of the IFNAR1 chain in procaryotic and eucaryotic cells has permitted the preparation of a series of recombinant soluble proteins corresponding to the extracellular domain of the IFNAR1 either as native isolated sequences or as fused proteins with the γ or κ chains of human IgG1 (Benoit et al., 1993, J. Immunol., 150, 707-716).

US Patent no. 5861258 relates to expression of the human Type 1-IFN receptor in non-human cells and use of such cells to screen for α-interferon agonists.

### Summary of the invention

Short peptides have now been derived from the IFNAR1 chain which are particularly effective Type 1-IFN antagonists. These peptides are believed to be derived from the binding site for human Type 1-IFN on its receptor.

In one aspect, the present invention provides a peptide selected from:
(i) FSSLKLNVY (Sequence ID no.1);
(ii) the peptide represented by Sequence ID no. 1 having an additional asparagine residue (N) at the C-terminus or an additional glutamic acid residue (E) at the N-terminus;
(iii) NFSSLKLNVYE (Sequence ID no. 2);
(iv) a peptide according to any of (i) to (iii) above in which the second serine residue from the N-terminus is substituted by an alanine residue.
These peptides are capable of inhibiting binding of a Type 1-IFN to the human IFN-R.

### Brief description of the figures

Figure 1 shows % Type 1-IFN binding to the IFN-R as presented by cultured Daudi cells in the presence of monoclonal antibody 64G12 or the same antibody together with an IFNAR1 chain-derived peptide or polypeptide (soluble IFNAR1 = amino acid residues 1 to 427 of the extracellular domain region of the IFNAR1 chain as reported by Uzé et al., 1990, Cell, 60, 224-234; IFNAR1 Pep. = Sequence ID no. 2);
Figure 2 shows results of ELISA binding tests of the peptide of Sequence ID no. 2 and modified versions thereof to monoclonal antibody 64G12.

### Detailed description of the invention

Peptides of the invention include peptides consisting of a portion of the native sequence of the IFNAR1. Particularly preferred of such peptides is the 9 mer of Sequence ID no. 1 corresponding to amino acid residues 88-97 of the IFNAR1 chain. Other preferred peptides of the invention corresponding to the native IFNAR1sequence are the 10 mers having an additional asparagine residue (N) at the C-terminus of Sequence ID no. 1 or an additional glutamic acid residue (E) at the N-terminus of Sequence ID no. 1 and the 11 mer NFSSLKLNVYE (Sequence ID no. 2).

The peptides of the invention corresponding to a native sequence of the IFNAR1 chain are able to specifically bind the anti-IFN-R monoclonal antibody 64G12, obtainable from the European Collection of Cell Structures (formally known as the European Collection of Animal Cell Cultures; ECACC) with reference to accession number 92022605 (hybridoma deposit made on 26.2.92 in the name of Laboratoire Europeen De Biotechnologie S.A. having its registered office at 28, Boulevard Camélinat-92233 Gennevilliers, France), or a functionally equivalent antibody to the IFNAR1 extracellular domain portion. Such antibodies which competitively bind with Type 1-IFN to the IFN-R are described in published International Application WO 93/20187. Typically, peptide analogues of the invention are also characterised by the ability to bind Mab 64G12 or an antibody which competitively binds with Mab 64G12 to the same epitope of the IFNAR1 chain.

A peptide of the invention as described above may be joined to an additional non-IFNARI sequence at the C- and/or N-terminus which does not abolish function as a Type 1-IFN antagonist.

A peptide of the invention may find application in the treatment or prophylaxis of a variety of diseased characterised by the abnormal or prolonged production of Type 1-IFN. Such diseases include but are not limited to alloy- or xeno-graft rejection, graft versus host disease, autoimmune diseases associated with abnormal prouction of Type 1-IFN including systemic lupus erythermatosus, rheumatoid arthritis, type 1 diabetes, psoriasis and multiple sclerosis and immune deficiency disorders associated with production of Type 1-IFN such as SCID and AIDS.

In a further aspect, the present invention provides a pharmaceutical composition comprising a peptide of the invention together with a pharmaceutically acceptable carrier or diluent. Such a pharmaceutical composition may be formulated in conventional manner.

In a still further aspect, the invention provides use of a peptide of the invention for the preparation of a composition for use in the treatment or prohylaxis of a disease selected from allograft or xenograft rejection, graft versus host disease, autoimmune diseases associated with abnormal production of Type 1-IFN and immune deficiency disorders associated with Type 1-IFN production. It will be appreciated that a peptide of the invention may be administered at doses conventional for peptide therapeutics.

A peptide of the invention may be administered via expression *in vivo* of a corresponding nucleic acid encoding the peptide. Thus, in yet another aspect, the present invention provides a nucleic acid capable of expressing a peptide or polypeptide of the invention in human cells for use as a Type 1-IFN antagonist. Such a nucleic acid may be a viral vector or a non-viral vector including such vectors packaged in a form for delivery of a nucleic acid of the invention to human cells. Thus, nucleic acids of the invention include viral vectors in a form suitable for viral vector therapy, for example, a recombinant retro virus, an adenovirus or attenuated influenza virus. Alternatively, a nucleic acid of the invention may be a non-viral vector, for example packaged into liposomes or into surfactin-containing vector delivery particles.

A peptide or polypeptide of the invention may be prepared by synthesis using conventional techniques or by expression of a nucleic acid in host cells. It may be produced by fragmentation of a longer sequence, e.g. a fusion polypeptide having an appropriate protease cleavage site for cleavage to obtain the desired peptide or polypeptide of the invention.

### The following examples illustrate the invention:

### EXAMPLES

### Example 1

### Inhibition of Type 1- IFN binding to the IFN-R on Daudi cells.

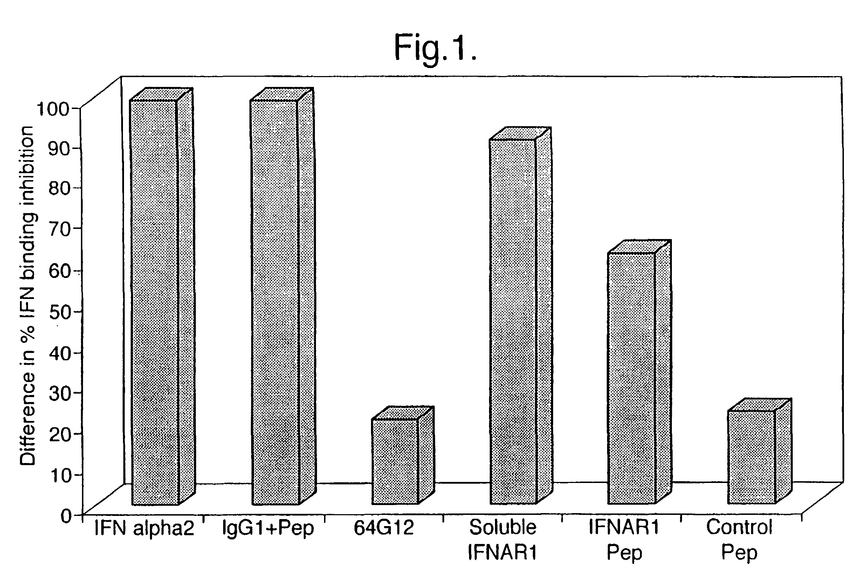

Cultures of exponentially growing Daudi cells (2 million cells/0.2 ml RPMI 1640 medium with 10% fetal calf serum) were treated with: Mab 64G12 (2µg), alone or together with: (i) a soluble affinity purified recombinant polypeptide corresponding to amino acids 1 to 427 of the extracellular domain region sequence of the IFNAR1 chain as reported by Uzé et al., 1990, Cell, 60, 224-234 (prepared as described in Benoit et al., 1993, J. Immunol., 150, 707-716 and Published International Application WO92/18626 and purified first on a NI-NTA agarose column (Qiagen) and subsequently eluted with 300 mM imidazole. The eluted partially purified soluble IFNAR1 was then applied to a 5.0 ml 64G12 Mab sepharose column and eluted with 0.1 M glycine, pH 2.8. The eluted IFNAR1 was pure as determined by SDS-PAGE under denaturing conditions) or (ii) the peptide of Sequence ID no. 2 (the 11 mer). This was followed by the addition of ¹²⁵I-labeled human IFN-α2 (iodination as described by Mogensen et al., 1981, Int. J. Cancer, 28, 575-582; 90000 cpm, 0.13 nM) and incubation for 2 hours at 4°C. The cells were then washed 3 times with culture medium containing 1% fetal calf serum and the cell pellet counted in a gamma counter. The % IFN binding is shown in Figure 1.

The 11 mer restored IFN-binding to a high degree in the presence of the anti-IFN-R monoclonal antibody 64G12. The same peptide does not affect the ability of a non-neutralising anti-IFN-R antibody (34F10), which recognises an epitope of the IFN-R distant from the ligand binding site, to bind to the IFN-R (Eid and Tovey, JICR 15, 205-211, 1995).

Both the 9 mer and 11mer were shown by ELISA to specifically bind Mab 64G12 as described in Example 2 below.

### Example 2

### Binding of peptides to Mab 64G12

The 11 mer (Sequence ID no.2) and a number of modified versions of that polypeptide derived by deletion or substitution were tested for ability to bind Mab 64G12 by ELISA. The mutated versions of Sequence ID no. 2 which were tested are listed in Table 1. The peptides were biotinylated at the N-terminus with a spacer sequence SGSG between the peptide and the biotin, i.e. biotin-SGSG-peptide.

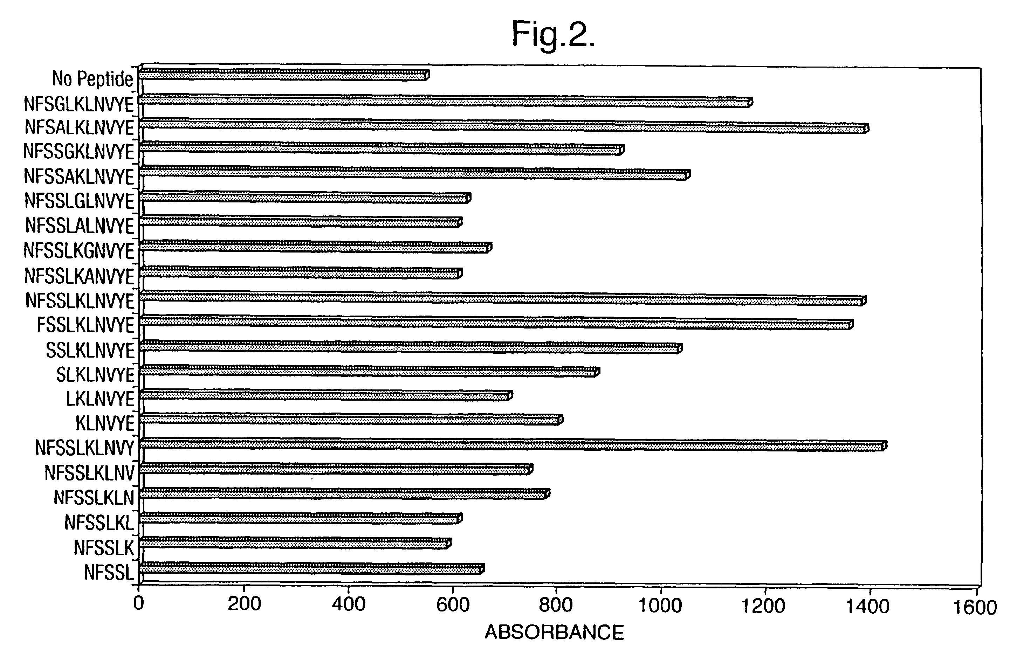

ELISA screening for ability to bind Mab 64G12 was carried out using Nunc Maxisorb plates coated with Streptavidin 5 µg/ml, 100 µl/well overnight at 37°C. The plates were then blocked with 200 µl/well of PBS containing 0.1% Tween 20, 1% sodium caseinate (CAST) for 1 hour at 20°C. Peptides were dissolved by adding 50 µl of DMSO and 0.6 ml of 40% acetonitrile to each tube. For peptide coating, 100 µl of PBS, 0.1% Tween 20 (PT) were added to each well, followed by 2 µl of each peptide solution (final peptide concentration 20 µM). After 30 mins, the monoclonal antibody was added at 1 µg/ml in PT for 1 hour at 20°C. The plates were washed and 100 µl/well of a 1/2000 dilution of horseradish peroxidase labeled goat anti-mouse IgG (H+L) in CAST plus 1% sheep serum (CASS) was added for 1 hour at 20°C. After washing, 100 µl of ABTS substrate was distributed to all wells. Absorbance was measured after 10 and 45 mins incubation on a plate reader using dual wavelength (405 and 490 nM) to correct for background. The results are shown in Figure 2.

The following conclusions can be drawn from Figure 2 as to the importance of positions in Sequence ID no. 2 for Mab 64G12 binding:
- the N-terminal asparagine (N) at position 1 is not critical for binding;
- the C-terminal glutamic acid (E) at position 11 is not critical for binding;
- Deletion of positions 2 (F) and 3 (S) reduces binding;
- Deletion of position 4 (S) or 5 (L) abolishes binding with the peptide KLNVYE showing only background binding;
- Substitution of the serine residue at position 4 with alanine or glycine does not substantially affect binding;
- Substitution of the leucine residue at position 5 by alanine or glycine significantly reduces binding;
- position 6 (K) and position 7 (L) are critical for binding;
- substitution of the lysine residue at position 6 or the leucine residue at position 7 by alanine or glycine totally inhibits binding;
- Loss of the tyrosine residue at position 10 dramatically reduces binding.

It can be anticipated that peptide analogues of Sequence ID no.1 or Sequence ID no. 2 which retain the ability to specifically bind Mab 64G12 or a functionally equivalent antibody will be effective Type 1- IFN antagonists.

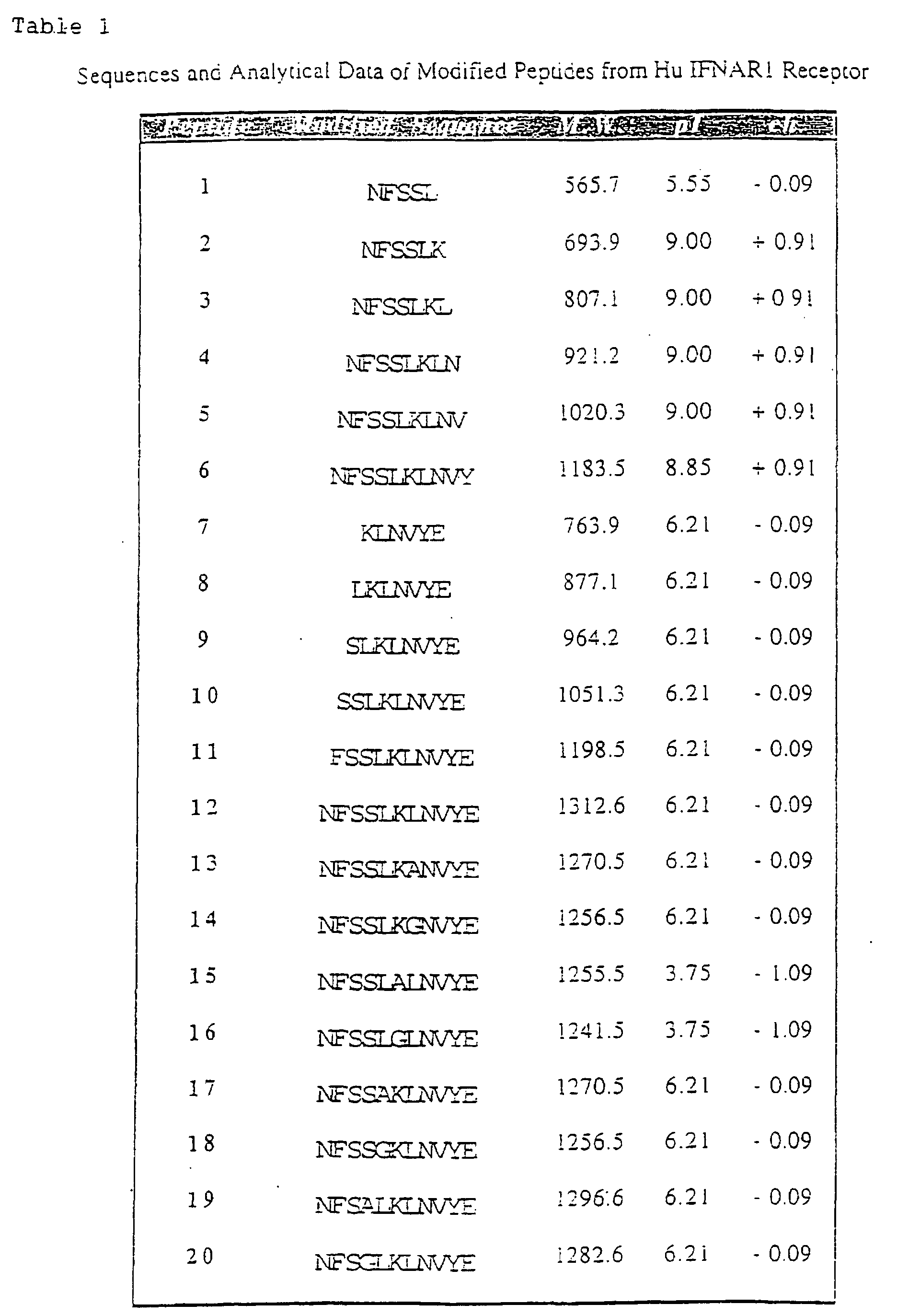

## Claims

1. A peptide selected from:
(i) FSSLKLNVY (Sequence ID no. 1);
(ii) the peptide represented by Sequence ID no. 1 having an additional asparagine residue (N) at the C-terminus or an additional glutamic acid residue (E) at the N-terminus;
(iii) NFSSLKLNVYE (Sequence ID no. 2);
(iv) a peptide according to any of (i) to (iii) above in which the second serine residue from the N-terminus is substituted by an alanine residue.

2. A peptide as claimed in claim 1 which is joined to an additional non-IFNAR1 sequence at the C- and/or N-terminus which does not abolish function as a Type 1-interferon (IFN) antagonist.

3. A peptide as claimed in claim 1 or claim 2 for use as a Type 1-interferon antagonist.

4. A pharmaceutical composition comprising a peptide as claimed in claim 1 or claim 2 together with a pharmaceutically acceptable carrier or diluent.

5. Use of a peptide as claimed in claim 1 or claim 2 for the preparation of a composition for use in the treatment or prophylaxis of a disease selected from allograft or xenograft rejection, graft versus host disease, autoimmune diseases associated with abnormal production of Type 1-IFN and immune deficiency disorders associated with Type 1-IFN production.

6. A nucleic acid capable of expressing in human cells a peptide as claimed in claim 1 or claim 2 for use as a Type 1-IFN antagonist.

7. A nucleic acid as claimed in claim 6 which is a viral vector or non-viral vector in a form for delivery of said nucleic acid to human cells.

## Patentansprüche

1. Peptid ausgewählt aus:
(i) FSSLKLNVY (Sequenz ID Nr. 1);
(ii) das Peptid der Sequenz ID Nr.1 mit einem weiteren Asparaginrest (N) am C-Terminus oder einem weiteren Glutaminsäurerest (E) am N-Terminus;
(iii) NFSSLKLNVYE (Sequenz ID Nr. 2);
(iv) Peptid gemäß einem der vorstehenden Punkte (i) bis (iii), wobei der zweite Serinrest vom N-Terminus durch ein Alaninrest substituiert ist.

2. Peptid gemäß Anspruch 1, das am C- und/oder N-Terminus mit einer weiteren nicht-IFNAR1-Sequenz verbunden ist, was die Funktion als Typ 1-Interferon (IFN)-Antagonist nicht aufhebt.

3. Peptid gemäß Anspruch 1 oder 2 zur Verwendung als Typ 1-Interferon-Antagonist.

4. Pharmazeutische Zusammensetzung, umfassend ein Peptid gemäß Anspruch 1 oder 2 zusammen mit einem pharmazeutisch verträglichen Träger oder Verdünnungsmittel.

5. Verwendung eines Peptids gemäß Anspruch 1 oder 2 zur Herstellung einer Zusammensetzung zur Verwendung bei der Behandlung oder Vorbeugung einer Krankheit ausgewählt aus der Gruppe, bestehend aus Allotransplantat- oder Xenotransplantatabstoßung, Transplantat-Wirt-Reaktion, Autoimmunkrankheiten, die mit anomaler Produktion von Typ 1-IFN in Zusammenhang stehen, und Immundefektstörungen, die mit Typ 1-IFN-Produktion in Zusammenhang stehen.

6. Nucleinsäure mit der Fähigkeit zum Exprimieren eines Peptids gemäß Anspruch 1 oder 2 in menschlichen Zellen zur Verwendung als Typ 1-IFN-Antagonist.

7. Nucleinsäure gemäß Anspruch 6, die ein viraler Vektor oder nicht-viraler Vektor ist, in einer Form zur Verabreichung der Nucleinsäure an menschliche Zellen.

## Revendications

1. Peptide choisi parmi :
(i) FSSLKLNVY (Séquence ID No. 1)
(ii) le peptide représenté par Séquence ID No. 1 ayant un résidu asparagine supplémentaire (N) à l'extrémité C-terminale ou un résidu acide glutamique (E) supplémentaire à l'extrémité N-terminale ;
(iii) NFSSLKLNVYE (Séquence ID No. 2) ;
(iv) un peptide selon l'un quelconque de (i) à (iii) ci-dessus, dans lequel le second résidu sérine à partir de l'extrémité N-terminale est remplacé par un résidu alanine.

2. Peptide tel que revendiqué dans la revendication 1, qui est joint à une séquence non-IFNAR1 supplémentaire à l'extrémité C- et/ou N-terminale qui n'annule pas la fonction d'antagoniste d'interféron (IFN) type 1.

3. Peptide tel que revendiqué dans la revendication 1 ou la revendication 2, pour une utilisation comme antagoniste d'interféron type 1.

4. Composition pharmaceutique comprenant un peptide tel que revendiqué dans la revendication 1 ou la revendication 2, avec un véhicule ou un diluant pharmaceutiquement acceptable.

5. Utilisation d'un peptide tel que revendiqué dans la revendication 1 ou la revendication 2 pour la préparation d'une composition destinée à être utilisée dans le traitement ou la prophylaxie d'une maladie choisie parmi un rejet d'allogreffe ou de xénogreffe, une maladie du greffon contre l'hôte, des maladies auto-immunes associées à une production anormale d'IFN type 1 et des affections de déficit immunitaire associées à la production d'IFN type 1.

6. Acide nucléique capable d'exprimer dans des cellules humaines un peptide tel que revendiqué dans la revendication 1 ou la revendication 2 pour une utilisation comme antagoniste d'IFN type 1.

7. Acide nucléique tel que revendiqué dans la revendication 6, qui est un vecteur viral ou un vecteur non viral sous une forme destinée à la délivrance dudit acide nucléique à des cellules humaines.
